# EUROPEAN PATENT SPECIFICATION

(11) **EP 1 444 969 B1**
(45) Date of publication and mention of the grant of the patent: **31.08.2011**
(21) Application number: 04002330.1
(22) Date of filing: 03.02.2004
(51) Int. Cl.: A61F 13/15, A61L 15/42, A61L 15/34, A61F 13/84

(54) **Absorbent article**
Absorbierender Artikel
Article absorbant

(30) Priority: 05.02.2003 JP 2003028881; 06.02.2003 JP 2003030028; 27.10.2003 JP 2003366582; 27.10.2003 JP 2003366587
(43) Date of publication of application: 11.08.2004
(73) Proprietor: KAO CORPORATION, Tokyo 103-8210 (JP)
(72) Inventor: Kasai, Takao, c/o Kao Corp. Research Laboratories, Haga-gun, Tochigi (JP); Sato Noriko, c/o Kao Corp. Research Laboratories, Haga-gun, Tochigi (JP)
(74) Representative: Vossius & Partner

(56) References cited:
- EP-A- 1 153 617
- EP-A- 1 166 766
- US-A- 4 790 836
- US-A1- 2002 120 241
- US-B1- 6 290 979

## Description

The present invention relates to absorbent articles such as diapers, sanitary napkins, incontinence pads, panty liners, sheets for vaginal discharges and the like containing a skin care agent.

In the case, for example, of diapers, wearing time is relatively longer and several excretions are observed during this time; therefore, there is a tendency of an extreme increase in humidity inside the diaper due to sweat and excrement. On easily sweating portions of the skin, in particular, there is a tendency of the formation of a rash due to such dampness. Therefore, a diaper is suggested suppressing the occurrence of rash by imparting a skin care effect.

For example, Japanese Patent Nos. 3217792 and 3217793 describe an absorbent product or absorbent ware having a top sheet or foot portion cuff carrying thereon an applied lotion agent containing an oily emollient agent and a fixing agent capable of fixing this emollient agent. Japanese Patent Application Laid-Open (JP-A) No. 2002-113039 describes an absorbent article in which a chemical having a predetermined efficacy is fixed at a predetermined site by a water-soluble sticker. JP-A No. 2002-200112 describes an absorbent article in which a chemical having a predetermined efficacy is kept at a predetermined site. WO 94/9757 describes a diaper having a porous sheet soaked with a skin lotion or a region soaked with a skin lotion.

When the technology described in Japanese Patent Nos. 3217792 and 3217793 is utilized in a diaper, an oily lotion agent moves to the skin of a wearer during wearing; therefore, adhesion of feces to the skin of a wearer can be suppressed, resulting also in the suppression of skin rash.

On the other hand, in the technology described in Japanese Patent Nos. 3217792 and 3217793, an emollient agent is fixed on the surface of a diaper with a fixing agent; consequently, the whole skin of a wearer in contact with a diaper cannot be coated with this emollient agent and it is difficult to suppress the contact of urine, which has higher flowability than feces, with the skin of a wearer. Therefore, the skin tends to manifest a rash due to contact with urine. Because of the same reason, skin also tends to manifest a rash when the emollient agent is utilized in a diaper during a wearing period in which urine is absorbed several times and in which humidity tends to increase therein due to absorption of urine, such as in diapers other than training pants.

When the technology described in JP-A Nos. 2002-113039 and 2002-200112 is applied to a diaper, a higher prevention effect can be obtained for rash of skin ascribable to contact with urine; however, this effect may lower when skin is injured by friction with a diaper or when a large amount of feces is secreted.

In the case of the diaper described in WO 94/9757, a skin lotion moves to the skin of a wearer directly after wearing; consequently, when a situation of easily getting a rash on skin arises, that is, when urination occurs or humidity in a diaper increases because of the absorption of urine, a sufficient skin care effect may not remain in some cases. Particularly with diapers worn for a long period of time, it is difficult to obtain a sufficient rash preventing effect.

The present inventionrelates to an absorbent article having at least a liquid-impermeable outer layer sheet, an absorber fixed on the outer layer sheet and a liquid-permeable surface sheet covering the absorber, wherein a water-soluble skin care agent and an oily skin care agent are applied on a part or all of a surface of the absorbent article to be contacted with the skin of a wearer while wearing, such that at least a part of the oily skin care agent is able to be transferred to the skin of a wearer when worn before the water-soluble skin care agent.
FIG. 1A is a top view schematically showing a diaper according to a first embodiment kept under a tense condition, and FIG. 1B is a bottom view schematically showing the diaper shown in FIG. 1A.
FIG. 2 is a top view schematically showing respective application regions of a water-soluble skin care agent and an oily skin care agent on the diaper shown in FIG. 1A and FIG. 1B.
FIGS. 3A to 3D are top views schematically showing respective application regions of a water-soluble skin care agent and an oily skin care agent in other embodiments.

The present invention relates to an absorbent article capable of suppressing rash of skin.

Rash of the skin can be suppressed utilizing both medicinal benefits of the oily skin care agent and the water-soluble skin care agent by applying the oily skin care agent and water-soluble skin care agent on an absorbent article so that at least a part of the oily skin care agent is present on the skin of a wearer after wearing the absorbent article before the water-soluble skin care agent is transferred to the skin of a wearer.

That is, in the initial wearing stage of an absorbent article, the oily skin care agent is allowed to be transferred to the skin of a wearer by contact with the skin of a wearer. Friction of the absorbent article and skin is lowered by the barrier effect of the oily skin care agent, to prevent injuring of skin by friction. Therefore, even if urine, feces, menstrual blood, vaginal discharge and the like (hereinafter, these are generically called excretions) are excreted, the occurrence of rash is suppressed as compared with the case of rash from injured skin. Further, by allowing the oily skin care agent to move to the skin of a wearer in the initial stage of wearing, direct contact of excretions with the skin can be suppressed by the barrier effect of the oily skin care agent even if there is excretion thereafter, and as a result, rash of skin ascribable to contact with excretions can be suppressed.

After wearing of an absorbent article, the water-soluble skin care agent under the oily skin care agent moves to the skin of a wearer owing to excretion to manifest its skin care effect. Consequently, rash of skin ascribable to contact with excretions is suppressed and, even if a rash occurs, this can be cured. Furthermore, the water-soluble skin care agent is actually effective at the stage of excretion and the skin care effect of the water-soluble skin care agent continues for a long period of time thereafter. Even if humidity in the absorbent article increases during this period, the occurrence of rash can be suppressed by this skin care effect. Even if rash occurs, this can be cured.

Therefore, by using the absorbent articles of the present invention, rash of skin can be suppressed as compared with conventional absorbent articles.

The diamide derivative of the above mentioned formula (II) is a diamide derivative described in WO 00/61097, and has medicinal benefits that the water retention ability and barrier function of a keratin layer are improved.

When this diamide derivative is applied on a predetermined surface of an absorbent article, that is, on a surface to be contacted with the skin of a wearer when wearing, this diamide derivative moves to the skin of a wearer when wearing the absorbent article, to function as a preferred oily skin care agent.

Therefore, the absorbent article can suppress rash of skin by the barrier effect of the oily skin care agent.

Embodiments of the absorbent article, which are non-limiting examples according to the present invention, will be described specifically below referring to the drawings.

### <First embodiment>

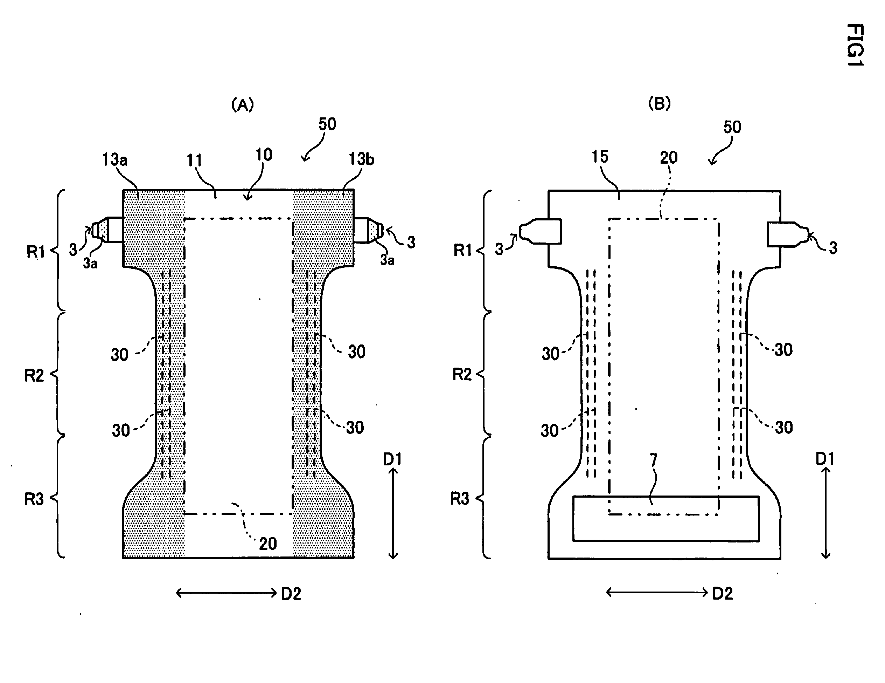

FIG. 1A is a top view schematically showing an absorbent article according to a first embodiment, and FIG. 1B is a bottom view schematically showing the absorbent article shown in FIG. 1A. In any view, the absorbent article is kept under a tense condition.

Herein, "top view" and "bottom view" referring to the absorbent article in the present specification mean a top view and a bottom view of an absorbent article placed so that its surface to be contacted with the body of a wearer when wearing faces upward. "An absorbent article is kept under a tense condition" means that the absorbent article is kept while applying tension along its width direction and longitudinal direction so that deformation ascribable to shrinkage of an elastic body provided on the absorbent article substantially disappears.

An absorbent article 50 as shown in the figures is an expansion type disposable diaper, and can be divided, for convenience, into three regions along the longitudinal direction shown by an arrow D1 in FIG. 1A and FIG. 1B (hereinafter, referred to as "longitudinal direction D1") . One region is a back side region R1 to be contacted mainly with from a buttock portion to a back portion, another region is a groin region R2 to be contacted mainly with the hypogastrium of a wearer, and further another region is an abdomen side region R3 to be contacted with the stomach.

For enhancing the fitting property to a femoral region of a wearer and improving its mounting property, the width of the groin region R2 is narrower than those of the other two regions R1 and R3, and an edge part of the back side region R1 by the groin region R2 side and an edge part of the abdomen side region R3 by the groin region R2 side both become narrower gradually toward the groin region R2.

When looking along the width direction shown by an arrow D2 in FIG. 1A and FIG. 1B (hereinafter, referred to as "width direction D2"), a region of the back side region R1 projecting toward the width direction D2 from the groin region R2 and a region of the abdomen side region R3 projecting toward the width direction D2 from the groin region R2 function respectively as side flaps to be contacted with the body side portion of a wearer.

On both left and right edges along the width direction D2 of the back side region R1, a couple of members 3 in the form of tape carrying a sheet fastener convex material 3a on one surface are fixed respectively. On the rear surface of the abdomen side region R3, a sheet fastener concave material 7 made of non-woven fabric is fixed, for example. In wearing the diaper 50, on left and right body side portions of a wearer, the member 3 in the form of tape is attracted up to the sheet fastener concave material 7 and the sheet fastener convex material 3a is connected to the sheet fastener concave material 7.

In the diaper 50, a liquid-permeable surface sheet 11 so placed as to cover the surface side of an absorber 20 described later, and liquid-impermeable side sheets 13a, 13b, are provided (hereinafter, surface sheet 11 and side sheets 13a, 13b are referred together to as "inner sheet 10", in some cases.). The side sheet 13a is connected to the left edge and the side sheet 13b is connected to the right edge, of the surface sheet 11, in FIG. 1A. In FIG. 1A, these side sheets 13a, 13b are indicated with shading for rendering the side sheets 13a, 13b comprehensible.

Outside of (on the rear surface of) an inner sheet 10, a liquid-impermeable outer layer sheet 15 formed using an organic polymer material such as polyethylene, polypropylene, polyester and the like is placed. The outline shape and size of the inner sheet 10 are approximately the same as the outline shape and size of the outer layer sheet 15, and by this inner sheet 10 and outer layer sheet 15, the outline shape on plane view and size of the diaper 50 excluding members 3 in the form of tape are substantially determined.

The absorber 20 for absorbing excretions is placed between the surface sheet 11 and the outer layer sheet 15, and covered on plane view with the surface sheet 11.

The absorber 20 is formed of an absorbent material layer made of, for example, an aggregate of hydrophilic fiber such as rayon, cellulose and the like, an aggregate obtained by performing hydrophilization treatment on synthetic fiber such as polyethylene fiber, polypropylene fiber and the like, an aggregate of the above mentioned hydrophilic fiber with the above mentioned hydrophilizated synthetic fiber, or those obtained by allowing these aggregates to carry a water-absorbent polymer, and the like, or formed by wrapping the above mentioned absorption material layer with one or more of paper, non-woven fabric, porous film and the like.

Between the inner sheet 10 and the outer layer sheet 15, elastic bodies 30 such as thread rubber, flat rubber and the like are placed on each of the left edge portion and the right edge portion, for preventing leakage of excretions via a groin of a wearer. These elastic bodies 30 are placed near the groin of a wearer in wearing, and shrink to a certain extent at least when the diaper 50 is in a natural condition, to form a gather. In the illustrated example, each two elastic bodies 30 are placed on each of the left edge portion and the right edge portion.

The great feature of the diaper 50 is that a water-soluble skin care agent and an oily skin care agent are applied in a predetermined pattern on the surface sheet 11. It is also one feature that the liquid-permeability of the surface sheet 11 is secured.

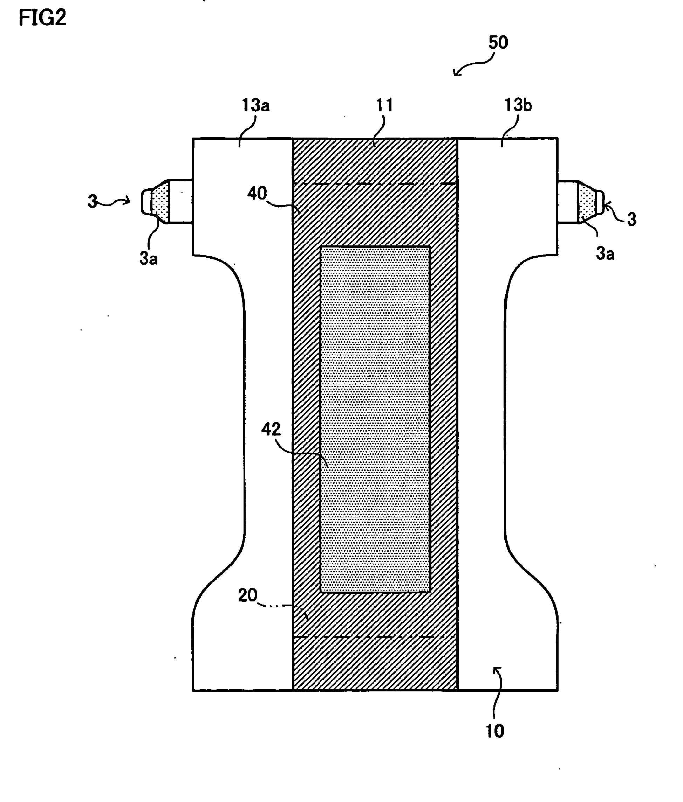

FIG. 2 is a top view schematically showing respective application regions of a water-soluble skin care agent and an oily skin care agent on the diaper 50 shown in FIG. 1A and FIG. 1B. As illustrated, the application region 40 with a water-soluble skin care agent approximately overlaps, on plane view, the surface sheet 11, and the application region 42 with an oily skin care agent is situated at the center of with width direction of the surface sheet 11, on plane view. The oily skin care agent is applied over the water-soluble skin care agent on the surface sheet 11. In FIG. 2, hatching is imparted to illustrate the application region 40 with a water-soluble skin care agent, and shading is imparted to illustrate the application region 42 with an oily skin care agent.

The above mentioned water-soluble skin care agent and oily skin care agent will be illustrated sequentially below.

### (A) Water-soluble skin care agent

The water-soluble skin care agent is preferably one which has water-solubility or water-dispersibility, suppresses the occurrence of rash and inflammation, and, when rash or inflammation occurs, suppresses progress of the rash and inflammation or relaxes the rash and inflammation.

Examples of such water-soluble skin care agents include (1) various plant extracts, (2) collagens, (3) humectants such as natural moisturizing factors (NMF), 1,3-butylene glycol, propylene glycol, dipropylene glycol, glycerin and the like, (4) keratin softening agents such as arginine, guanidine derivatives (for example, organic acid salts (for example, succinate, glycolate, lactate, malate, citrate, tartarate and the like) of 2-(2-hydroxyethoxy)ethylguanine), and the like, (5) antiphlogistics such as guaiazulene, tannin, gallic acid derivatives and the like.

In view of solubility in water, skin care effect and cost, it is preferable to use, as the water-soluble skin care agent, plant extracts or natural moisturizing factors (NMF). Specific examples of the above mentioned plant extract include oat extract, sea weed (Fucus) extract, citrus extract, hamamelis (Hamamelis virginiana) extract, burnet extract, hiba arborvitae extract, aloe extract, phellodendron bark powdered extract, field horsetail extract, chamomilla redutita extract, eucalyptus extract, peach leaf extract and the like. Particularly, a higher skin care effect can be obtained by using citrus extract, hamamelis extract, hiba arborvitae extract, aloe extract, phellodendron bark powdered extract, eucalyptus extract or peach leaf extract.

When a highly water-absorbing polymer is used as a material of absorption bodies, it is preferable that degradations such as deterioration of the absorbing performance of the absorbing polymer and the like are not caused by a water-soluble skin care agent. For this, it is preferable to use, as a water-soluble skin care agent, citrus extract, hamamelis extract, hiba arborvitae extract, aloe extract, phellodendron bark powdered extract, eucalyptus extract or peach leaf extract, particularly, citrus extract, hamamelis extract or hiba arborvitae extract.

Plant extracts can be obtained by, for example, drying the whole bodies of various plants or one or more parts of the leaf, bark, root and branch thereof, grinding them alternatively without drying, and extracting them under normal temperature or heating by a solvent, or extracting them using an extraction instrument such as a Soxhlet extractor and the like. When a 1, 3-butylene glycol extract is used as a plant extract, solubility in water increases, and additionally, necessity for explosion? protection disappears even if the heating treatment is conducted in a process of producing fiber or diapers. Further, when it is applied on a surface sheet or fiber as a material of the surface sheet, decrease in the absorbing performance of an absorber constituting a diaper is suppressed.

Usually, a plant extract contains solvent components and water used for extraction in addition to effective components. In this condition, the amount of effective components (amount excluding evaporation solvent components and water) of a plant extract may preferably be generally from about 0.001 to 5 wt%. When 1,3-butylene glycol is used as a solvent component for extraction, the sum amount of effective components of a plant extract and 1,3-butylene glycol may be in the above mentioned range. The amount of effective components in a plant extract is preferably from about 0.001 to 10 wt% based on the amount of solvent components.

It is preferable that the above mentioned water-soluble skin care agent is fixed on a predetermined part (surface sheet 11 in the embodiment shown in FIG. 2) of a diaper under a condition wherein the water-soluble skin care agent is releasable with the application of water, and particularly, it is preferable that the above mentioned water-soluble skin care agent is fixed on a diaper under a condition wherein the skin care agent is dissolvable or dispersible in excretions by contact with the excretions. When the water-soluble skin care agent is present on a diaper under such conditions, this water-soluble skin care agent easily moves to the skin of a wearer upon its exposure to excretion and therefore, the effect is easily manifested.

Herein, "fixed under condition releasable with water" used for a water-soluble skin care agent in the present specification means that this water-soluble skin care agent has a high water-solubility or water-dispersibility. For example, "fixed under condition releasable with water" is established if, when a sheet on which a water-soluble skin care agent is applied is soaked in water (liquid temperature: 25°C) in an amount of 10 times of the weight of this sheet, the water-soluble skin care agent is substantially completely dissolved or dispersed.

It is advantageous that a water-soluble skin care agent can move from an absorbent article to the skin of a wearer using water as the main means, or a water-soluble skin care agent may move to the skin of a wearer using an auxiliary means other than water.

Fixing of a water-soluble skin care agent to an absorbent article can be conducted, for example, by applying a liquid coating (agent) containing a water-soluble skin care agent on a predetermined position of the surface sheet 11 or inner sheet 10 or a sheet as a material for these sheets, and drying the liquid.

In this example, a surfactant is used together in addition to a water-soluble skin care agent. A water-soluble skin care agent is fixed stably on a surface sheet until excretion releases the skin care agent; therefore, it is possible to suppress movement of a water-soluble skin care agent - to the skin while the oily skin care agent is being transferred directly to the skin of a wearer after wearing. When excretion occurs, a large proportion of the water-soluble skin care agent moves to the side of the absorber together with the surfactant, and in this phenomenon, a part of the water-soluble skin care agent contacts the skin of a wearer.

Additionally, simultaneous use of a surfactant has merits also in hydrophilicity, repeating absorbability of an absorber in an absorbent article, or rust prevention for facilities which produce non-woven fabric and absorbent articles, and non-woven fabric processability and the like.

Therefore, even if excretions come into contact with skin, the water-soluble skin care agent protects the skin, and the occurrence of rash is suppressed or its symptoms are relaxed. Further, since movement of a water-soluble skin care agent to the skin occurs at the time of excretion, even if, for example, humidity in a diaper is increased by the absorption of urine and this condition lasts for a long period of time, it is possible to allow the skin care effect of the water-soluble skin care agent to continue sufficiently. As a result, the skin of a wearer is protected by this skin care effect, and the occurrence of rash is suppressed or its symptoms are relaxed.

Fixing of a water-soluble skin care agent to an absorbent article can also be conducted, when the subject on which the water-soluble skin care agent is to be fixed is a non-woven fabric, by previously fixing a water-soluble skin care agent to fiber constituting the non-woven fabric and producing the non-woven fabric using this fiber as a material.

In this case, fixing of a water-soluble skin care agent can be conducted, for example, by preparing a liquid coating containing a water-soluble skin care agent and further containing a surfactant and fiber treating agent if necessary, and adhering this liquid coating to the above- mentioned fiber by a method such as dipping, spray coating, roll coating and the like, then, drying the fiber.

The surface sheet 11 is often formed using hydrophobic fiber as the main material so that excretions easily permeate. into an absorber; therefore, it is particularly preferable that the above mentioned liquid coating contains a surfactant. By allowing a surfactant to be contained in the above-mentioned liquid coating, fixing of a water-soluble skin care agent to the surface of a hydrophobic fiber becomes more stable. Additionally, simultaneous use of a surfactant has merits also in hydrophilicity, repeating absorbability of an absorber in an absorbent article, or rust prevention for facilities for producing non-woven fabric and absorbent articles, and non-woven fabric processability and the like.

As this surfactant, any anionic surfactant, cationic surfactant and nonionic surfactant can preferably be used. Specific examples thereof include polyoxyethylene alkyl sulfate metal salts, alkyl sulfate metal salts, polyoxyethylene alkyl phosphate metal salts, alkyl phosphate metal salts, fatty diethanol amide, fatty sorbitan esters (may be any of mono, di andtriester), alkyl glucoside, polyglycerin fatty esters, alkyl succinate metal salts and the like. As the above mentioned metal salts, for example, sodium salts and potassium salts can be used.

The surfactant may be used alone or in a combination of two or more surfactants. In view of hydrophilicity, repeating absorbability of an absorber in an absorbent article, or rust prevention for facilities for producing non-woven fabric and absorbent articles, and non-woven fabric processability and the like, it is particularly preferable to use, as the surfactant, one or more of polyoxyethylene alkyl phosphate metal salts, alkyl phosphate metal salts, fatty diethanol amide, fatty sorbitan esters, alkyl glucoside, and alkyl succinate metal salts.

From the standpoint of fixing stably a water-soluble skin care agent to the surface of the hydrophobic fiber, it is preferable to use a hydrophobic surfactant and a hydrophilic surfactant in admixture. For example, when two or more nonionic surfactants are used in admixture, HLB (hydrophilicity lipophilicity balance) can be used as an index. When a hydrophilic surfactant having a HLB of 8 or more, particularly 10 or more, and a hydrophobic surfactant having a HLB of 6 or less, particularly 5 or less, are used in admixture at a mixing ratio of hydrophilic surfactant/hydrophobic surfactant of 90/10 to 30/70(wt%), fixing of a water-soluble skin care agent with a surface sheet produced using hydrophobic fiber as the main material is stabilized, and even if an oily skin care agent is released by contact with the skin of a wearer directly after wearing of an absorbent article, the water-soluble skin care agent tends to remain on the surface sheet. Additionally, a water-soluble skin care agent is easily released from the surface of the surface sheet after exposure to excretion.

In addition to the surfactants described above, antistatic agents, antioxidants, pH regulators, smoothing agents, emulsifiers, antimicrobial agents, antifungal agents, aromatics and the like can be formulated.

In the case of fixing of a water-soluble skin care agent by any method, this water-soluble skin care agent may be continuously adhered in the form of a membrane or layer on the surface of the fixation subject, or maybe adhered discontinuously, for example, in the form of particles or a discontinuous film.

The suitable fixing amount of a water-soluble skin care agent differs depending on the kind of water-soluble skin care agent used, and can be appropriately selected in a range so as not to deteriorate skin. When the fixing amount of a water-soluble skin care agent is too large, the skin care effect is saturated, and therefore, it is preferable to determine the upper limit of this fixing amount in view of the skin care effect and economy. For example, when a plant extract is used as a water-soluble skin care agent and this water-soluble skin care agent is fixed on the surface of the fiber, the fixing amount preferably can be from 0.01 to 5 wt%, and in particular, more preferably from about 0.5 to 2 wt%.

For fixing a water-soluble skin care agent on the surface of the fiber under a condition releasable by water, it is preferable to add a surfactant to the water-soluble skin care agent and allow the surfactant to be adhered on the surface of the fiber together with 1,3-butylene glycol. Though 1,3-butylene glycol can be used as a water-soluble skin care agent itself as described above, it is particularly preferable, when a plant extract is used as a water-soluble skin care agent, to use the 1,3-butyleneglycol as a solvent for fixation of the plant extract to the surface of the fiber.

### (B) Oily skin care agent

As the oily skin care agent, those having efficacies such as protection, cure and the like for the skin of a wearer can be used without specific restriction, and for example, those used as an emollient agent in the field of cosmetics can be used.

Specific examples of the oily skin care agent include liquid paraffin, silicone oil, animal and vegetable oil (olive oil, jojoba oil, safflower oil, squalane and squalene, and the like), monoglyceride, diglyceride, triglyceride, aliphatic ether (myristyl-1,3-diemthyl butyl ether, palmityl-1,3-dimethyl butyl ether, stearyl-1,3-dimethyl butyl ether, palmityl-1,3-methyl propyl ether, stearyl-1,3-methyl propyl ether and the like) , and bisabolol, isostearyl cholesterol ester and the like.

Further, paraffin wax, C₁₂ to C₂₂ fatty acids, C₁₂ to C₄₄ fatty ethers, C₁₂ to C₂₂ fatty alcohols, Vaseline, metal soap (magnesium stearate, and the like), sucrose fatty ester, cyclodextrin fatty ester, silicone resin, alkyl silicone, silicone, fatty sorbitan ester (may be any of monoester, diester and triester) , polyoxyethylene fatty sorbitan ester (may be any of monoester, diester and triester), and emollient agents described in Japanese Patent No. 3217792, JP-A Nos. 2002-113039 and 2002-200112, WO 94/9757, and the like can also be used as the above mentioned oily skin care agent.

Further, it is also possible to use, as an oily skin care agent, a diamide derivative of the following formula (I) or the following formula (III) described in WO 00/61097: 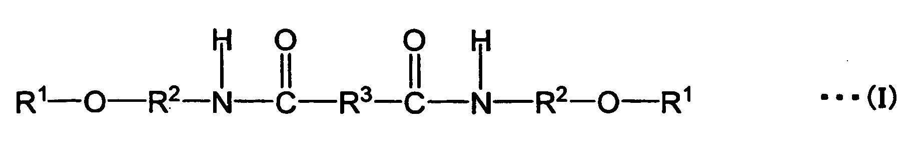 (wherein, R¹ represents a linear or branched hydrocarbon group having 1 to 22 carbon atoms optionally substituted with a hydroxy group and/or alkoxy group, R² represents a linear or branched divalent hydrocarbon group having 1 to 12 carbon atoms, and R³ represents a linear or branched divalent hydrocarbon group having 1 to 42 carbon atoms.) 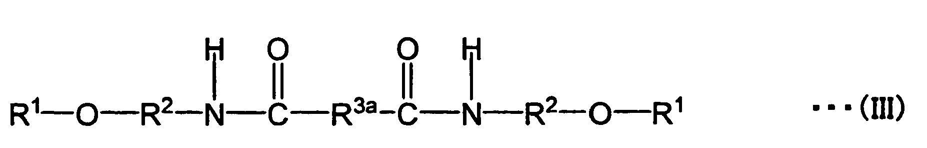 (wherein, R¹ represents a linear or branched hydrocarbon group having 1 to 22 carbon atoms optionally substituted with a hydroxy group and/or alkoxy group, R² represents a linear or branched divalent hydrocarbon group having 1 to 12 carbon atoms, and R^{3a} represents a linear or branched alkylene group having 11 to 42 carbon atoms or an alkenylene group having 1 to 4 double bonds) .

An oily skin care agent plays mainly a role that it is transferred to the skin of a wearer when initially worn, exerts a barrier effect, and suppresses direct contact of excretions to the skin of a wearer, and by this, suppresses the occurrence of rash by suppressing skin stimulation by excretions. Further, it manifests a function that it weakens friction of skin with a diaper to decrease skin stimulus and injuring of skin, softens skin after being absorbed percutaneously, and enhances the barrier function of skin. Therefore, the oily skin care agent is applied on a predetermined region of a sheet or diaper on which a water-soluble skin care agent is applied, and fixed.

As this oily skin care agent, it is preferable to use those having a viscosity at 36°C of 5000 mPa·s or less, more preferably from 200 to 5000 mPa·s, even more preferably from 500 to 2000 mPa·s, or those of which viscosity is controlled to obtain such viscosity.

By using an oily skin care agent having a viscosity as described above, the oily skin care agent can be effectively transferred to the skin of a wearer. That is, when the viscosity of an oily skin care agent is 5000 mPa·s or less, the property oftransferring to skin becomes extremely excellent, and it is not necessary to use an oily skin care agent in a large amount for realizing a predetermined rash suppressing effect. Further, the influence on absorbability decreases. When the viscosity of an oily skin care agent is 200 mPa.s or more, movement of an oily skin care agent to other sites can be effectively prevented even in the case of preservation (storage) for a long period of time of an absorbent article before wearing; as a result, a predetermined skin care effect is obtained effectively.

"Viscosity of an oily skin care agent" in the present invention means the viscosity measured as described below.

### <Method of measuring viscosity of oily skin care agent>

Measurement is conducted using a tuning fork type vibration mode viscometer (VIBRO NISCOMETERCJV5000 (manufactured by A&D Company Limited) is typically used in this embodiment). An oily skin care agent heated previously at about 100°C is gradually cooled to room temperature, and viscosity at a predetermined temperature is measured.

The oily skin care agent used in the present invention preferably shows smaller dependency of viscosity on temperature. Specifically, preferable are those having a viscosity at 36°C within the above mentioned range and a viscosity at 80°C of from 50 to 200 mPa·s. When dependency of viscosity on temperature is small, an oily skin care agent can be uniformly applied easily on a surface sheet, and it is stably fixed on a surface sheet even if some temperature change occurs; consequently, there is scarce tendency of the occurrence of absorption disturbance by penetration or diffusion of an oily skin care agent in an absorber. Further, a two-stage skin care effect tends to be manifested in which an oily skin care agent moves first to the skin of a wearer, then, a water-soluble skin care agent moves to the skin of the wearer.

Application of an oily skin care agent on an absorbent article or its material can be conducted, for example, by putting the oily skin care agent into a tank of an applicator, and if necessary, heating this to enhance its flowability, then, coating this on a predetermined position on an absorbent article or its material.

Methods of coating an oily skin care agent include a dice coater method, slot spray method, curtain spray method, melt blown method, spiral spray method, gravure method, bead method and the like.

When a method of spraying with air is used as in a spray method, an oily skin care agent is converted into fine particles or mist and sprayed on the surface of an application subject. For this reason, an oily skin care agent is continuously provided macroscopically on a surface sheet, while microscopically, the agent is distributed discontinuously on the surface of an application subject.

As a result, it becomes possible to secure the liquid permeability of a surface sheet and effectively utilize the absorbing ability of an absorber while performing a skin care effect in a wider range. Therefore, application of an oily skin care agent by a method of spraying with air is suitable for obtaining a desired absorbent article. Of course, even in the case of applying a method of spraying with air, an oily skin care agent can be applied under predetermined patterns such as stripes, lattices and the like as described in the description of the application according to a dice coater method described later.

In a dice coater method, an oily skin care agent is applied in the form of a sheet or layer to an application subject as compared with other methods; therefore, the absorbing ability of an absorber lowers in some cases depending on the kind and application amount of an oily skin care agent or on its application position on the sheet or layer. Therefore, when an oily skin care agent is applied by applying this method to a surface sheet covering an absorber or to a sheet used as its material, it is preferable to make contrivance to suppress the coating ratio of an absorber with an oily skin care agent on plane view, such as application in the form of a stripe along the longitudinal direction D1 or width direction D2 (see FIG. 1A), application in the form of lattices, and the like.

From the standpoint of obtaining a surface sheet 11 having practical liquid-permeability and on which an oily skin care agent is applied, it is preferable to select the application amount and application pattern of an oily skin care agent so that the liquid-permeability of the surface sheet 11 in a region on which an oily skin care agent has been applied is preferably 10 seconds or less, more preferably 3 seconds or less in terms of liquid permeation time on the surface sheet 11 measured based on the definition of JIS L 1096 (Japan Industry Standard ).

It is preferable to select the application amount of an oily skin care agent in view not only of an influence on the absorbing ability of an absorber but also of feeling to the touch and any skin care effect. For example, when an oily skin care agent is applied continuously in the form of a film or layer on a surface sheet, contact area with the skin of a wearer spreads; consequently, the amount of movement of an oily skin care agent to skin increases and skin is easily coated over broader range, to enhance a skin care effect, while, the absorbing ability of an absorber may be decreased and a sticky feeling is generated in some cases.

In view of the influence on the absorbing ability of an absorber, skin care effect, feeling to the touch and the like, the application amount of an oily skin care agent on an absorber (meaning application amount in a region on an absorber on plane view, of regions on which an oily skin care agent has been applied) is preferably in the range from 0.05 to 10 g/m², more preferably in the range from 0.1 to 5 g/m², depending on the kind and application pattern of an oily skin care agent used. This application amount can be determined from a difference in weight of an application subject before and after application of an oily skin care agent.

If necessary, two kinds of application methods may be used together in one absorbent article. For example, a dice coater method may be adopted for the absorbent article' s position which contacts with the wearer's buttock area for which a higher skin care effect is required, and a spray method may be adopted around the absorbent article' s portion wherein the wearer urinates for which both an absorbing ability and a skin care effect are required.

In the case of the diaper 50 shown in FIG. 2, the water-soluble skin care agent and oily skin care agent described above are applied in this order on the surface sheet 11; as a result, in the initial stage of wearing of the diaper 50, the oily skin care agent is transferred to the skin of a wearer by contact with the skin of a wearer, and because of the barrier effect of the oily skin care agent, friction of the diaper 50 with skin is decreased, and injuring of the skin by friction is prevented. For this reason, even if excretion occurs thereafter, the occurrence of rash is suppressed as compared with the case of injured skin. Further, even if feces excretion occurs, direct contact of the skin with feces is suppressed by the oily skin care agent that have been transferred to the skin of a wearer; consequently, rash of skin ascribable to contact with feces is suppressed.

After wearing of the diaper 50, the water-soluble skin care agent under the oily skin care agent moves to the skin of a wearer in the time of excretion to manifest its skin care effect; as a result, rash of skin ascribable to contact with urine and feces is suppressed. At the stage of excretion, the skin care effect of the water-soluble skin care agent is substantially manifested; consequently, the skin care effect of the water-soluble skin care agent is easily allowed to continue over a longer period of time thereafter, and even if humidity in the diaper 50 increases during this period, the occurrence of rash can be suppressed owing to this skin care effect.

Conventionally, it has been suggested to suppress rash by applying any of the oily skin care agents or water-soluble skin care agents in a sufficient amount to a diaper. However, the present inventors and the like have found that rash occurs in some cases even in these types of diapers. For example, listed are (1) a case of wearing a diaper for a long period of time, (2) a case of higher air temperature and room temperature, (3) a case of use of a baby stroller and child car seat when going outdoors, which increases close adherence of a diaper with the skin of a wearer, and (4) a case of frequent feces excretion of a wearer or such case caused by diarrhea.

Any of these cases has a condition in which there is an increase in humidity in a diaper and maceration of the skin of a wearer due to this increase in humidity; further, injuring of the skin of a wearer due to contact with excretions (stimulation) or friction with a diaper or a baby wiping sheet, occur remarkably. For this reason, the occurrence of rash tends to occur, and once occurred, it tends to become severe. Then, it is necessary to conduct a double approach which are preventing the occurrence of rash and curing or relaxing occurred rash, for solving these cases. Although it has been contemplated to increase the application amount of the above mentioned skin care agent, when the application amount of an oily skin care agent is increased, problems occur such as decrease in water-permeability (liquid-permeability) of a surface sheet, stickiness and the like. On the other hand, even if the amount of a water-soluble skin care agent is increased excessively, the end effect is saturation of the surface sheet.

Then, co-use of a water-soluble skin care agent and an oily skin care agent was contemplated. In this case, since both of the skin care agents are poor in compatibility, it is considered to mix both the skin care agents with a surfactant and water to form an emulsion system and to apply this on a sheet.

According to the study of the present inventors, however, it has been found that a sufficient rash preventing effect cannot be obtained only with this condition. Although not wanting to be limited by theory, as a result of the analysis, the grounds for this have been guessed to be a fact that the skin of a wearer placed for a long period of time in a closed space in a diaper is under a severe environment, and by only one movement or transfer of a skin care agent to skin, it is difficult to coat the whole skin contacting a diaper with a skin care agent, and its skin care effect decreases with the lapse of time.

Therefore, in the present invention, a non-emulsification system was adopted in which an oily skin care agent is provided on a water-soluble skin care agent already provided. It has been found that in applying to a surface sheet, by allowing liquid coating containing a water-soluble skin care agent to contain a surfactant, a preferable condition can be easily formed in which a water-soluble skin care agent easily transfers after transfer of an oily skin care agent to skin, and permeability of the excretion through the sheet and into the absorber can also be secured.

With the above mentioned diaper 50, rash of the skin of a wearer can be suppressed even when the diaper is worn for a long period of time or in the case of diarrhea of a wearer.

Preferably, each sheet used as a material of an absorbent article is formed of non-woven fabric in many cases. In applying (fixing) the above mentioned diamide derivative or water-soluble skin care agent on non-woven fabric in one embodiment, fiber constituting the non-woven fabric is not particularly restricted, and may be semi-synthetic fiber such as rayon, Lyocell and the like, synthetic fiber containing a single fiber made of a thermoplastic resin such as polyethylene, polypropylene, polyethylene terephthalate, polyacrylic acid, polyamide and the like, and complex fiber of a core-sheath type or side by side type, and the like, and also, natural fiber such as pulp, cotton and the like.

Fiber constituting non-woven fabric may be used singly or in combination, and each fiber may be in the form of either a long fiber or short fiber. Further, fineness of fiber is also not particularly restricted, and can be appropriately selected depending on the application, grade and the like of the intended diaper; however, the fineness of fiber used in the above mentioned inner sheet and surface sheet is preferably in the range from about 0.5 to 8.9 dtex, more preferably from about 1 to 5.6 dtex.

The absorbent article of the present invention is not limited to the above mentioned diapers (disposable diapers), and can be applied likewise also to other absorbent articles such as sanitary napkins, incontinence pads, panty liners, vaginal discharge sheets and the like.

### <Second embodiment>

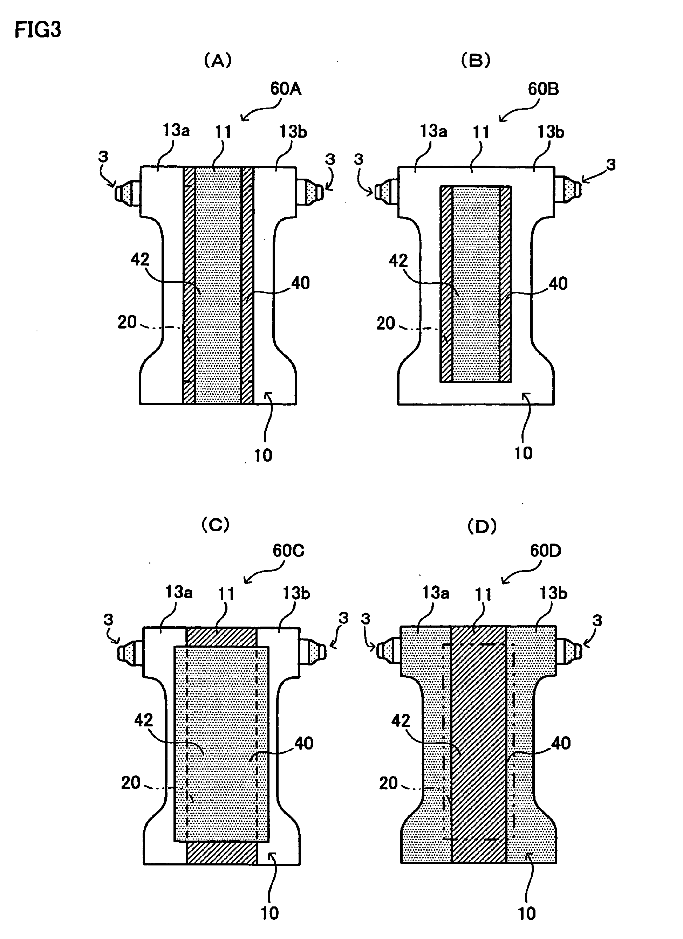

FIGS. 3A to 3D are top views schematically showing the application regions of a water-soluble skin care agent and oily skin care agent, respectively, of diapers 60A to 60D which are other embodiments.

In the diaper 60A shown in FIG. 3A, an application region 40 with a water-soluble skin care agent approximately overlaps a surface sheet 11 on plane view, and an application region 42 with an oily skin care agent ranges from one end to another end along the longitudinal direction of the surface sheet 11 at the center of the width direction of the surface sheet 11 on plane view. A region in which the application region 40 overlaps the application region 42 is on the surface sheet 11.

In the diaper 60B shown in FIG. 3B, an application region 40 with a water-soluble skin care agent approximately overlaps an absorber 20 on plane view, and an application region 42 with an oily skin care agent ranges from one end to another end along the longitudinal direction of the absorber 20 at the center of the width direction of the absorber 20 on plane view. A region in which the application region 40 overlaps the application region 42 is on the absorber 20.

In the diaper 60C shown in FIG. 3C, an application region 40 with a water-soluble skin care agent approximately overlaps a surface sheet 11 on plane view, and an application region 42 with an oily skin care agent is wider than an absorber 20 along the width direction, and has approximately the same length with an absorber 20 along the longitudinal direction. The absorber 20 is covered with the region 42 on plane view, and on the absorber 20, the application region 40 and the application region 42 overlap.

In the diaper 60D shown in FIG. 3D, an application region 40 with a water-soluble skin care agent ranges from one end to another end along the longitudinal direction of the surface sheet 11 and is narrower slightly than the width of an absorber 20, and there is a region of no application of a water-soluble skin care agent on an edge part of the absorber 20 along the width direction. The application region 42 with an oily skin care agent covers over the entire inner sheet 10. On the absorber 20, there is a region in which the application region 40 and the application region 42 overlap. In FIG. 3D, drawing of the application region 42 on the application region 40 is abbreviated for convenience.

The constitutions of these diapers 60A to 60D are the same as the constitution of the diaper 50 in the first embodiment excepting the application region 40 with a water-soluble skin care agent or the application region 42 with an oily skin care agent; therefore, those common to the constituent members shown in FIG. 2 are endowed with the same reference marks as those used in FIG. 2 and explanation thereof is omitted.

The above-mentioned diapers 60A to 60D perform the same technological effect as that of the diaper 50 in the first embodiment. When an oily skin care agent is applied also on an elastic membrane 30 (see FIG. 1) to be contacted with parts around the groin of a wearer when wearing, the occurrence of inflammation and the like on the skin of a wearer by friction with a gather formed while wearing by the elastic member 30 can be prevented, suitably.

Though the diapers 50, 60A to 60 D in the above mentioned embodiments are all expansion type diapers, the diaper of the present invention may also be of a pants type.

An inner sheet 10 in an expansion type diaper and a surface sheet 11 in a pants type diaper are formed of a non-woven fabric in may cases. In application (fixation) of a water-soluble skin care agent and an oily skin care agent on non-woven fabric, fiber constituting the non-woven fabric is not particularly restricted, and may be semi-synthetic fiber such as rayon, Lyocell and the like, synthetic fiber containing a single fiber made of a thermoplastic resin such as polyethylene, polypropylene, polyethylene terephthalate, polyacrylic acid, polyamide and the like, and complex fiber of core-sheath type or side by side type, and the like, and also, natural fiber such as pulp, cotton and the like.

Fiber constituting non-woven fabric may be used singly or in combination, and each fiber may be either a long fiber or short fiber. Further, fineness of fiber is also not particularly restricted, and can be appropriately selected depending on the application, grade and the like of the intended diaper; however, the fineness of fiber used in the above mentioned inner sheet and surface sheet is often in the range from about 0.5 to 8.9 dtex, particularly from about 1 to 5.6 dtex.

In any of the expansion type diaper and pants type diaper, a standing guard (standing gather) is provided in some cases to prevent leaking from the groin of a wearer or regions around the groin. When a standing guard is provided, a water-soluble skin care agent and/or oily skin care agent can be applied on a predetermined position of the standing guard or a predetermined position of a sheet used as a material of the standing guard.

In addition to a water-soluble skin care agent, a friction reducing agent optionally can also be adhered to a diaper for the purpose of suppressing injuring of skin. As the friction reducing agent, a crystal in the form of a plate made of an organic substance and, organic fine particles can be used. As the above mentioned crystal in the form of a plate, listed are, for example, acylated taurine metal salts (lauroyltaurine calcium salt, lauroyl-β-alanyl calcium), disteary ether, zinc sodium cetylphosphate, N-ε-lauroyl-L-lysine and the like, and as the above mentioned organic particles, for example, silicone beads, nylon beads, chitosan beads and the like.

In the water-soluble skin care agent application region, sweat suppressing agents such as aluminum hydroxy chloride, aluminum-zirconium salt, zinc p-phenolsulfonate and the like can also be applied, if necessary.

### <Related technology>

The great feature of the diaper 50 in this related technology is that a diamide derivative of the above mentioned formula (II) is applied on a surface to be contacted with the skin of a wearer when wearing.

The above mentioned diamide derivative moves to the skin of a wearer on wearing of a diaper 50, and performs functions that (1) it suppresses direct contact of excretions with the skin of a wearer to suppress skin stimulus by the excretions, (2) it weakens friction of skin with a diaper to decrease skin stimulus by a diaper, and (3) it improves the water retention ability and barrier function of a keratin layer after percutaneous absorption, working as an oily skin care agent. Therefore, in the diaper 50, rash of skin is suppressed.

As the above mentioned diamide derivative, it is preferable to use those having a viscosity at 36°C of 5000 mPa·s or less, more preferably from 200 to 5000 mPa·s, even more preferably from 500 to 2000 mPa·s, or those of which viscosity is controlled to obtain such viscosity, from the standpoint of efficient movement of the diamide derivative to the skin of a wearer.

By using a diamide derivative having viscosity as described above, the oily skin care agent can be effectively moved to the skin of a wearer. That is, when the viscosity of a diamide derivative is 5000 mPa·s or less, a property of moving to skin becomes extremely excellent, and there is no necessity to use an oily skin care agent in a large amount for realizing a desired rash suppressing effect. Further, an influence on absorbability decreases. When the viscosity of an oily skin care agent is 200 mPa·s or more, movement of the above mentioned diamide derivative to other sites can be effectively prevented even in the case of preservation for a long period of time of an absorbent article before wearing; as a result, a predetermined skin care effect is obtained effectively.

"Viscosity of a diamide derivative" in the present invention was measured by the same measuring method as used for the viscosity of an oily skin care agent in the above mentioned first embodiment.

The diamide derivative used in the present invention preferably shows smaller dependency of viscosity on temperature. Specifically, preferable are those having a viscosity at 36°C within the above mentioned range and a viscosity at 80°C of from 50 to 200 mPa·s. When dependency of viscosity on temperature is small, a diamide derivative can be uniformly applied easily on a sheet, and it is stably fixed on a sheet even if a some temperature change occurs; consequently, there is scarce tendency of occurrence of absorption disturbance by penetration or diffusion of a diamide derivative into an absorber.

Application of a diamide derivative on an absorbent article or its material can be conducted by the same application method as for an oily skin care agent in the above mentioned first embodiment.

It is preferable to select the application amount of a diamide derivative in view not only of an influence on an absorbing ability of an absorber but also of feeling to the wearer and a skin care effect. For example, when a diamide derivative is applied continuously in the form of a film or layer, the contact area with the skin of a wearer spreads; consequently, the amount of movement of a diamide derivative to the skin increases and skin is easily coated over a broader range, to enhance a skin care effect, while, the absorbing ability of an absorber may be decreased easily and a sticky feeling is generated in some cases.

In view of a skin care effect, feeling and the like, the application amount of a diamide derivative (meaning application amount in a region on which a diamide derivative is actually applied) is preferably in the range from about 0.05 to 20 g/m², more preferably in the range from about 0.1 to 10 g/m². Particularly in the case of application on a surface sheet, a range of from 0.1 to 5 g/m² is even more preferable. This application amount can be determined from a difference in weight of an application subj ect before and after application of a diamide derivative.

If necessary, two kinds of application methods preferably can be used together in one absorbent article. For example, a dice coater method may be adopted in a position of contact of the absorbent article with the buttock area for which a higher skin care effect is required and a spray method may be adopted around a urination portion of the absorbent article for which both an absorbing ability and a skin care effect are required.

When a diamide derivative is applied also on an absorber, the water absorption speed (JIS L 1096 (Japan Industry Standard) , water-absorption, water-absorbing speed, method A (dropping method) ) of a surface sheet can preferably be made to 10 seconds or less by controlling its application amount in the above mentioned range. Therefore, an absorbent article having a practical absorbing ability can be obtained.

The diamide derivative may be used singly or in combination of two or more. If necessary, it can be used together with other oily skin care agents. As the other oily skin care agent, those having efficacies such as protection, cure and the like for the skin of a wearer can be used without specific restriction, and for example, those used as an emollient agent in the field of cosmetics can be used.

As the above mentioned other oily skin care agent, those oily skin care agents exemplified in the first embodiment can be used.

Though the absorbent article of the above mentioned embodiment is an expansion type diaper, the absorbent article of the present invention may also be a pants type diaper. In any of the expansion type diapers and pants type diapers, it is preferable to apply a diamide derivative of the formula (II) locally at least on an absorber or apply to coat the whole body of an absorber on plane view, from the standpoint of suppressing rash of skin.

The absorbent article of the present invention is not limited to diapers and can be applied also to other absorbent articles such as sanitary napkins, incontinence pads, panty liners, vaginal discharge sheets and the like.

Each sheet used as a material of an absorbent article is formed of a non-woven fabric in many cases, and sheets of the same embodiment as shown in the above mentioned first embodiment can be used.

A surface sheet in a sanitary napkin is formed of a liquid-permeable porous film in some cases. Even if the surface sheet is formed of a porous sheet, it is possible to obtain the same technological effect as for each diaper in the abovementioned embodiments by applying a diamide derivative of the formula (II) locally on this surface sheet or so as to cover the surface sheet, if necessary, using a water-soluble skin care agent together.

Irrespective of the kind of a material of an application subj ect, by using a diamide derivative of the formula (II) together with silicone, it becomes easy to reduce friction of an absorbent article in one preferred embodiment with the skin of a wearer and it becomes easy to suppress stickiness caused by a diamide derivative. Further, silicone can function as a releasing agent in applying a diamide derivative. The amount of silicone which may be used is preferably from about 0.1 to 5 parts by weight based on 100 parts by weight of a diamide derivative of the formula (II). As the silicone, those of any condition and nature such as a liquid and solid can be used, and in the case of a liquid, those having a viscosity of from about 5 to 10000 mPa·s, further from 10 to 1000 mPa·s at 36°C are suitable. When silicone is used together, it is preferable that the above mentioned diamide derivative is provided as a mixture with silicone on an absorbent article, and it is preferable that the viscosity of this mixture is about 5000 mPa·s or less, more preferably from 200 to 5000 mPa·s, even more preferably from 500 to 2000 mPa·s.

Irrespective of use or no use of silicone, a friction reducing agent may be adhered to an absorbent article for the purpose of suppressing injuring of skin. As the friction reducing agent, a crystal in the form of plate made of an organic substance and, organic fine particles can be used. As the above mentioned crystal in the form of a plate, listed are, for example, acylated taurine metal salts (lauroyltaurine calcium salt, lauroyl-β-alanyl calcium), disteary ether, zinc sodium cetylphosphate, N-ε-lauroyl-L-lysine and the like, and as the above mentioned organic particles, for example, silicone beads, nylon beads, chitosan beads and the like.

If necessary, sweat suppressing agents such as aluminum hydroxy chloride, aluminum-zirconium salt, zinc p-phenolsulfonate and the like may also be applied.

The present invention is not limited to the above mentioned embodiments. The above mentioned embodiments are only examples, and any absorbent articles having substantially the same constitution as the technological idea described in the claims of the invention and performing the same action and effect are included in the technological range of the present invention.

### EXAMPLES

The following examples will specifically explain particular embodiments of the present invention. The examples are given solely for the purpose of illustration and are not to be construed as limitations of the present invention.

### (Example 1)

An expansion type diaper was produced having the same constitution as that of the diaper 50 in the first embodiment (see, FIG. 1A, FIG. 1B, and FIG. 2).

In this procedure, used as the surface sheet 11 in the inner sheet 10 was a non-woven fabric having basis weight of 30 g/m² obtained by using a core-sheath type complex fiber having a core component made of polypropylene and a sheath component made of polyethylene and having a fineness of 3.3 dtex. Before the formation of the non-woven fabric, a liquid coating prepared by mixing hamamelis extract (1,3-butylene glycol extraction liquid) as a water-soluble skin care agent, polyoxyethylene sorbitan monolaurate (HLB = 13.3) which is a hydrophilic surfactant, sorbitan tristearate (HLB = 2.1) which is a hydrophobic surfactant, monoalkylphosphate potassium salt, an antistatic agent, a smoothing agent was applied on the above mentioned core-sheath type complex fiber. In preparing the liquid coating, the addition amount of sorbitan tristearate was the same as the addition amount of polyoxyethylene sorbitan monolaurate.

After formation of the non-woven fabric, a diamide derivative of the following formula (IV) was applied as an oily skin care agent on a predetermined region. This diamide derivative is one of the diamide derivatives of the abovementioned formula (I):

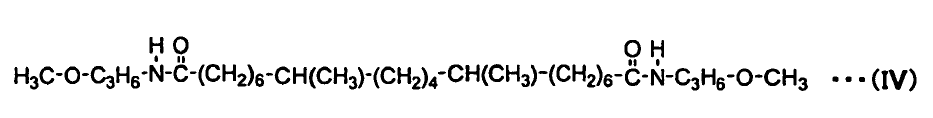

The application amount (fixation amount) of the above mentioned hamamelis extract is 0.07 wt%, the application amount (application amount at applied regions) of the above mentioned diamide derivative is 1.0 g/m², and the viscosity at 36°C of the applied diamide derivative is 1800 mPa·s. Application of the diamide derivative was conducted under a temperature of 80°C using a spray coater.

As the side sheets 13a, 13b on the inner sheet 10, a non-woven fabric having a weight of 20 g/m² formed of polypropylene fiber was used, and as the outer layer sheet 15, used was a liquid-impermeable gas-permeable film obtained by uniformly mixing linear low density polyethylene and calcium carbonate, then, extruding the mixture via a dice to obtain a film, and performing mono-axial drawing treatment on this film, and having a moisture permeability (according to JIS Z 0208 - Japan Industry Standard...) of 4800 g/m²·24 hours. As the absorber 20, a body was used obtained by mixing a high water-absorbing polymer and pulp at a weight ratio of 50:50 to obtain an absorbing material and covering this with a mounting.

### (Comparative Example 1)

A diaper was produced in the same manner as in Example 1 except that an oily skin care agent was not coated on the non-woven fabric for the surface sheet 11.

### <Ability evaluation>

### (1) Diaper rash preventing ability

The diapers produced respectively in Example 1 and Comparative Example 1 were used by 10 monitors, and the relative occurrence frequency of rash was measured when the rash occurrence frequency in Comparative Example 1 was 1. When this value is smaller, the rash preventing ability is higher as compared with the diaper in Comparative Example 1. The results are shown in Table 1.

### (2) Liquid-permeability of the surface sheet

Regarding the diapers obtained respectively in Example 1 and Comparative Example 1, the liquid permeation time through the surface sheet was measured by water absorption, water absorbing speed, and method A (dropping method) defined in JIS L 1096 (Japan Industry Standard...). When this permeation time is shortened, the absorbing speed is faster; that is, the liquid-permeability is higher. The results are shown in Table 1.

Evaluation of liquid-permeability was conducted at a place on which an oily skin care agent was present.

**Table 1**

| | Water-soluble skin care agent | Oily skin care agent | Rash occurrence frequency *1 | Liquid permeation time through surface sheet |
|---|---|---|---|---|
| Example 1 | Hamamelis extract | Diamide derivative of the formula (III) | 0.5 | About 1 second |
| Comparative Example 1 | Hamamelis extract | - | 1 | About 1 second |

| | | | | |
|---|---|---|---|---|
| *1: it shows relative value when the rash occurrence frequency on the diaper produced in Comparative Example 1 is 1 | | | | |

As apparent from Table 1, the diaper produced in Example 1 was excellent in rash preventing ability as compared with that produced in Comparative Example 1. Further, it was shown that even if an oily skin care agent is used (Example 1), the liquid-permeability through a surface sheet is high like the case of using only a water-soluble skin care agent (Comparative Example 1).

### (Example 2)

### <Test 1 for ability of suppressing occurrence of erythema>

Using inside parts of forearms (both arms) of five healthy adults, a patch test was conducted using a predetermined combination of the water-soluble skin care agent and oily skin care agent shown in Table 2, and a test was conducted on the ability of suppressing the occurrence of erythema. As the oily skin care agent of sample No. 2, a monoamide compound of the following formula (V) (viscosity at 36°C was 1000 mPa·s) was used.

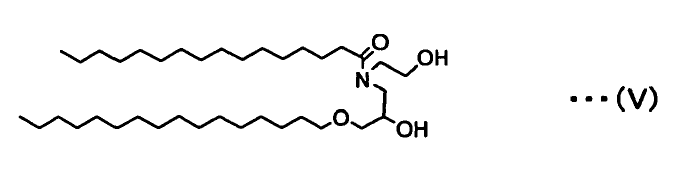

First, a predetermined position inside of the forearm was cleaned with an acetone/ether (1:1) solution, and a non-woven fabric on which an oily skin care agent had been applied at a proportion of 1. 0 g/m² was placed onto the forearm under a pressure of 4.4 kPa. Thereafter, an adhesive tape for the patch test soaked with a water-soluble skin care agent and ammonia 1. 5 mass% physiological saline (100 µl) was pasted on over the same position for 1.5 hours. After removal of the adhesive tape, it was left for 30 minutes, and the condition of the occurrence of erythema was confirmed by visual determination at four stages (0: no erythema, 1: slightly red, 2: somewhat red, 3: red). The average values (level of severity of erythema) of the condition of the occurrence of erythema are shown in Table 2.

**Table 2**

| Sample No. | Water-soluble skin care agent | Oily skin care agent | Level of severity of erythema |
|---|---|---|---|
| 1 | Hamamelis extract | Diamide of the formula (IV) | 1.6 |
| 2 | Hamamelis extract | Monoamide of the formula (V) | 1.2 |
| 3 | Hamamelis extract | Vaseline | 1.2 |
| 4 | Hamamelis extract | None | 2.0 |
| 5 | None | Diamide of the formula (IV) | 1.8 |

As apparent from Table 2, it was conformed that the occurrence of erythema is more effectively suppressed in the case of using also an oily skin care agent (sample No. 1) as compared with the case of use of only a water-soluble skin care agent (sample No. 4), conforming the erythema occurrence suppressing effect of the oily skin care agent. Further, it was confirmed that the occurrence of erythema is more effectively suppressed in the case of using an oily skin care agent and water-soluble skin care agent in combination, an embodiment of the present invention (sample Nos. 1 to 3) as compared with the case of use of an oily skin care agent or a water-soluble skin care agent singly (sample No. 4 and sample No. 5).

### (Example 3)

### <Test 2 for ability of suppressing occurrence of erythema>

Using the inside parts of forearms of thirteen healthy adults, a test was conducted on the ability of suppressing the occurrence of erythema by a water-soluble skin care agent shown in Table 3. First, a predetermined position inside of the forearm was cleaned with an acetone/ether (1:1) solution. Thereafter, an adhesive tape for patch test soaked with ammonia 2 mass% physiological saline (15 µl) and 15 µl of various plant extract 1% solutions (1,3-butylene glycol extraction solution)(since peach leaf extract is dry powder, only peach leaf extract: 0.01%) shown in Table 3 was pasted for 3 hours. After removal of the adhesive tape, it was left for 150 minutes, and condition of the occurrence of erythema was confirmed by visual determination at four stages. The ratio (B/A) of (A) a visual determination result of erythema in the case of using an adhesive tape soaked only with ammonia and (B) a visual determination result of erythema in the case of using each water-soluble skin care agent was measured for each member, and the number of adults showing this value less than 1 was divided by all 13 to give a value as an anti-inflammation score. The results are shown in Table 3. It is shown that when the anti-inflammation score is high, the effect of suppressing the occurrence of erythema is high.

**Table 3**

| Sample No. | Water-soluble skin care agent | Anti-inflammation score |
|---|---|---|
| 6 | Hamamelis extract | 0.42 |
| 7 | Peach leaf extract | 0.16 |
| 8 | Citrus extract | 0.50 |
| 9 | Eucalyptus extract | 0.18 |
| 10 | Hiba arborvitae extract | 0.38 |

As apparent from Table 3, it was confirmed that in the case of using any water-soluble skin care agent, the occurrence of erythema is effectively suppressed as compared with the case of no use thereof, confirming that any agent has a high skin care effect. Therefore, it is supposed that the same skin care effect is performed even in the case of using these water-soluble skin care agents instead of the hamamelis extract in Example 1 and Example 2 described above.

### (Effect of the Invention)

According to the present invention, a diaper is provided capable of suppressing rash of skin. Therefore, according to the present invention, it becomes easy to provide a diaper having improved safety.

## Claims

1. An absorbent article (50) comprising at least a liquid-impermeable outer layer sheet (15), an absorber (20) fixed on the outer layer sheet(15) and a liquid-permeable surface sheet (11) covering the absorber (20), **characterized in that** a water-soluble skin care agent and an oily skin care agent being applied in this order on a part or all of a surface of the absorbent article to be contacted with the skin of a wearer while wearing, such that at least a part of the oily skin care agent is able to be transferred to the skin of a wearer when worn before the water-soluble skin care agent is able to be transferred to the skin of wearer.

2. The absorbent article (50) according to Claim 1, wherein on at least the surface sheet (11), the water-soluble skin care agent is adhered together with a surfactant, and the permeation time of liquid through the surface sheet (11) measured based on the definition of JIS L 1096 is 10 seconds or less.

3. The absorbent article(50) according to Claim 1 or 2, wherein the oily skin care agent is placed so that a part of the water-soluble skin care agent is exposed to the side of the skin of the wearer.

4. The absorbent article (50) according to any one of Claims 1 to 3, wherein the viscosity at 36°C of the oily skin care agent is 5000 mPa.s or less.

5. The absorbent article (50) according to any one of Claims 1 to 4, wherein the water-soluble skin care agent is a plant extract and the oily skin care agent is a diamide derivative of the following formula (I) 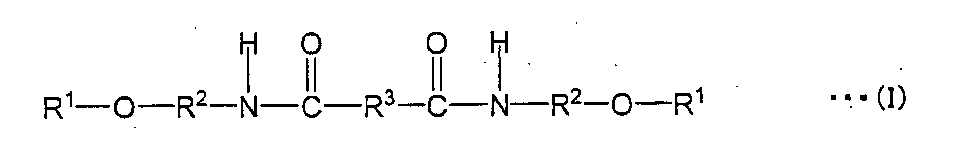 wherein, R¹ represents a linear or branched hydrocarbon group having 1 to 22 carbon atoms optionally substituted with a hydroxy group and/or alkoxy group, R² represents a linear or branched divalent hydrocarbon group having 1 to 12 carbon atoms, and R³ represents a linear or branched divalent hydrocarbon group having 1 to 42 carbon atoms.

6. The absorbent article (50) according to any one of Claims 1 to 5, wherein the oily skin care agent is applied on the surface sheet (11), and the application amount of the oily skin care agent on regions on which the oily skin care agent has been applied on the surface sheet (11) is in the range from 0.05 to 10 g/m².

7. The absorbent article (50) of claim 1, wherein the oily skin care agent is a diamide derivative of the following formula (II) 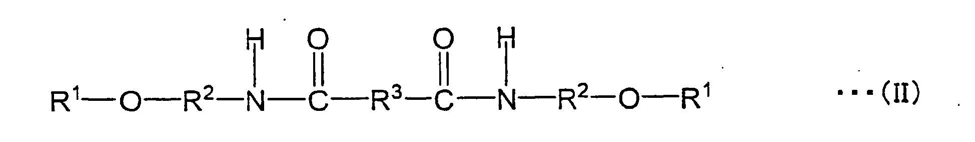 wherein, R¹ represents a linear or branched.hydrocarbon group having 1 to 22 carbon atoms optionally substituted with a hydroxy group and/or alkoxy group, R² represents a linear or branched divalent hydrocarbon group having 1 to 12 carbon atoms, and R³ represents a linear or branched divalent hydrocarbon group having 1 to 42 carbon atoms, a linear or branched alkylene group having 11 to 42 carbon atoms or an alkenylene group having 1 to 4 double bonds.

8. A use of the absorbent particle (50) according to any one of Claims 1 to 7 in suppressing rash of skin.

## Patentansprüche

1. Absorbierender Artikel (50) mit mindestens einer flüssigkeitsundurchlässigen Außenschichtbahn (15), einem auf der Außenschichtbahn (15) fixierten Absorber (20) und einer flüssigkeitsdurchlässigen Oberflächenbahn (11), die den Absorber (20) bedeckt, **dadurch gekennzeichnet, dass** ein wasserlösliches Hautpflegemittel und ein öliges Hautpflegemittel in dieser Reihenfolge auf einen Teil oder die Gesamtheit einer mit der Haut eines Trägers beim Tragen in Kontakt zu bringenden Oberfläche des absorbierenden Artikels so aufgetragen sind, dass mindestens ein Teil des öligen Hautpflegemittels zur Haut eines Trägers beim Tragen übertragen werden kann, bevor das wasserlösliche Hautpflegemittel zur Trägerhaut übertragen werden kann.

2. Absorbierender Artikel (50) nach Anspruch 1, wobei auf mindestens der Oberflächenbahn (11) das wasserlösliche Hautpflegemittel zusammen mit einem oberflächenaktiven Stoff haftet und die Permeationszeit von Flüssigkeit durch die Oberflächenbahn (11) in der Messung auf der Grundlage der Festlegung nach JIS L 1096 höchstens 10 Sekunden beträgt.

3. Absorbierender Artikel (50) nach Anspruch 1 oder 2, wobei das ölige Hautpflegemittel so platziert ist, dass ein Teil des wasserlöslichen Hautpflegemittels zur Hautseite des Trägers freiliegt.

4. Absorbierender Artikel (50) nach einem der Ansprüche 1 bis 3, wobei die Viskosität des öligen Hautpflegemittels bei 36 °C höchstens 5000 mPas·s beträgt.

5. Absorbierender Artikel (50) nach einem der Ansprüche 1 bis 4, wobei das wasserlösliche Hautpflegemittel ein Pflanzenextrakt ist und das ölige Hautpflegemittel ein Diamidderivat mit der folgenden Formel (I) ist: 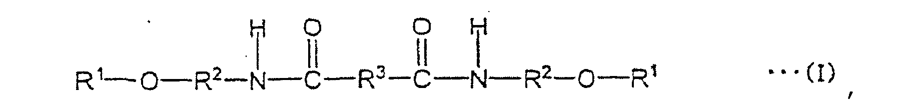 wobei R¹ eine gerad- oder verzweigtkettige Kohlenwasserstoffgruppe mit 1 bis 22 Kohlenstoffatomen, optional substituiert durch eine Hydroxylgruppe und/oder Alkoxylgruppe, darstellt, R² eine gerad- oder verzweigtkettige zweiwertige Kohlenwasserstoffgruppe mit 1 bis 12 Kohlenstoffatomen darstellt und R³ eine gerad- oder verzweigtkettige zweiwertige Kohlenwasserstoffgruppe mit 1 bis 42 Kohlenstoffatomen darstellt.

6. Absorbierender Artikel (50) nach einem der Ansprüche 1 bis 5, wobei das ölige Hautpflegemittel auf die Oberflächenbahn (11) aufgetragen ist und die Auftragsmenge des öligen Hautpflegemittels auf Regionen, auf die das ölige Hautpflegemittel auf die Oberflächenbahn (11) aufgetragen wurde, im Bereich von 0,05 bis 10 g/m² liegt.

7. Absorbierender Artikel (50) nach Anspruch 1, wobei das ölige Hautpflegemittel ein Diamidderivat mit der folgenden Formel (II) ist: 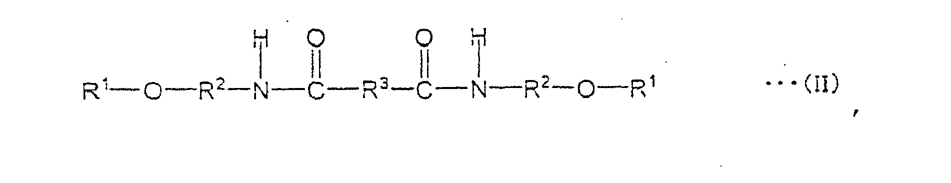 wobei R¹ eine gerad- oder verzweigtkettige Kohlenwasserstoffgruppe mit 1 bis 22 Kohlenstoffatomen, optional substituiert durch eine Hydroxylgruppe und/oder Alkoxylgruppe, darstellt, R² eine gerad- oder verzweigtkettige zweiwertige Kohlenwasserstoffgruppe mit 1 bis 12 Kohlenstoffatomen darstellt und R³ eine gerad- oder verzweigtkettige zweiwertige Kohlenwasserstoffgruppe mit 1 bis 42 Kohlenstoffatomen, eine gerad- oder verzweigtkettige Alkylengruppe mit 11 bis 42 Kohlenstoffatomen oder eine Alkenylengruppe mit 1 bis 4 Doppelbindungen darstellt.

8. Verwendung des absorbierenden Artikels (50) nach einem der Ansprüche 1 bis 7 beim Unterdrücken von Hautausschlag.

## Revendications

1. Article absorbant (50) comprenant au moins une feuille de couche extérieure, imperméable aux liquides (15), un absorbant (20) fixé à la feuille de couche extérieure (15) et une feuille superficielle, perméable aux liquides (11) recouvrant l'absorbant (20), **caractérisé en ce qu'**un agent de soins de la peau soluble dans l'eau et un agent de soins de la peau huileux sont appliqués, dans cet ordre, sur toute ou partie d'une surface de l'article absorbant destinée à entrer en contact avec la peau de l'utilisateur lors du port de l'article, pour qu'au moins une partie de l'agent de soins de la peau huileux puisse être transférée sur la peau de l'utilisateur lors du port de l'article, avant que l'agent de soins de la peau soluble dans l'eau ne puisse être transféré sur la peau de l'utilisateur.

2. Article absorbant (50) selon la revendication 1, dans lequel sur au moins la feuille superficielle (11), l'agent de soins de la peau soluble dans l'eau est collé avec un tensioactif et le temps de perméation du liquide à travers la feuille superficielle (11), mesuré selon la définition de JIS L 1096, est de 10 secondes ou moins.

3. Article absorbant (50) selon la revendication 1 ou 2, dans lequel l'agent de soins de la peau huileux est placé de façon qu'une partie de l'agent de soins de la peau soluble dans l'eau soit exposée du côté de la peau de l'utilisateur.

4. Article absorbant (50) selon l'une quelconque des revendications 1 à 3, dans lequel la viscosité à 36 °C de l'agent de soins de la peau huileux est de 5000 mPa·s ou moins.

5. Article absorbant (50) selon l'une quelconque des revendications 1 à 4, dans lequel l'agent de soins de la peau soluble dans l'eau est un extrait végétal et l'agent de soins de la peau huileux est un dérivé de diamide répondant à la formule (I) suivante 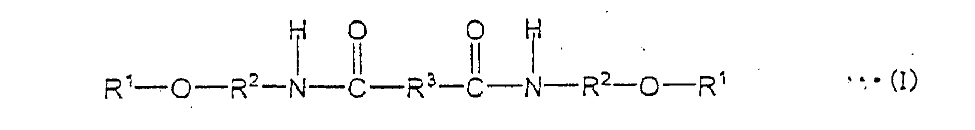 dans laquelle, R¹ représente un groupe hydrocarbure linéaire ou ramifié ayant de 1 à 22 atomes de carbone éventuellement substitué par un groupe hydroxy et/ou un groupe alcoxy, R² représente un groupe hydrocarbure divalent linéaire ou ramifié ayant de 1 à 12 atomes de carbone, et R³ représente un groupe hydrocarbure divalent linéaire ou ramifié ayant de 1 à 42 atomes de carbone.

6. Article absorbant (50) selon l'une quelconque des revendications 1 à 5, dans lequel l'agent de soins de la peau huileux est appliqué sur la feuille superficielle (11), et la quantité appliquée d'agent de soins de la peau huileux sur les zones où l'agent de soins de la peau huileux a été appliqué sur la feuille superficielle (11) est dans la plage de 0,05 à 10 g/m².

7. Article absorbant (50) selon la revendication 1, dans lequel l'agent de soins de la peau huileux est un dérivé de diamide répondant à la formule (II) suivante 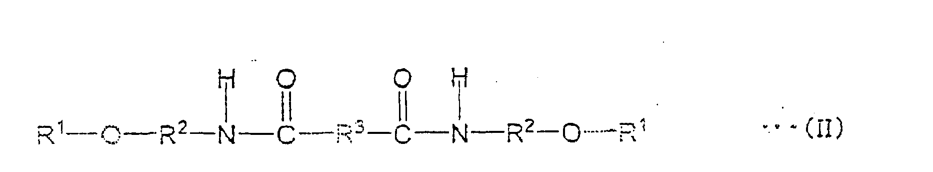 dans laquelle, R¹ représente un groupe hydrocarbure linéaire ou ramifié ayant de 1 à 22 atomes de carbone éventuellement substitué par un groupe hydroxy et/ou un groupe alcoxy, R² représente un groupe hydrocarbure divalent linéaire ou ramifié ayant de 1 à 12 atomes de carbone, et R³ représente un groupe hydrocarbure divalent linéaire ou ramifié ayant de 1 à 42 atomes de carbone, un groupe alkylène linéaire ou ramifié ayant de 11 à 42 atomes de carbone ou un groupe alkénylène ayant de 1 à 4 doubles liaisons.

8. Utilisation de l'article absorbant (50) selon l'une quelconque des revendications 1 à 7 pour supprimer les éruptions cutanées.
